# DEMANDE DE BREVET EUROPEEN

(11) **EP 1 095 616 A2**
(43) Date de publication de la demande: **02.05.2001**
(21) Numéro de dépôt: 00122643.0
(22) Date de dépôt: 18.10.2000
(51) Int. Cl.: A61B 5/107

(54) **Dispositif de mesure anthropometrique**

(30) Priorité: 30.10.1999 CH 198399
(71) Demandeur: Winkenbach, Laurent M., 2400 Le Locle (CH); Ferraroli, Alessandro M., 6963 Pregassona (CH)
(72) Inventeur: Winkenbach, Laurent, 2400 Le Locle (CH); Ferraroli, Alessandro, 6963 Pregassona (CH)
(74) Mandataire: Gresset, Jean

(57) **Abrégé**

Dispositif de mesure anthropométrique pour la détermination de caractéristiques relatives à l'ossature d'un individu (12). Ce dispositif comporte:
- un podium (10) sur lequel ledit individu (12) prend place,
- des marques (48) disposées sur le corps dudit individu (12), en des endroits correspondant à la position des articulations,
- des moyens de prise de vue (14) de type numérique pour enregistrer les informations visibles concernant l'individu placé sur le podium,
- un ordinateur (52) muni d'un programme informatique agencé de manière à déterminer la structure de l'ossature et à calculer les dimensions d'un outil devant être manipulé par ledit individu.

Le tout est agencé de manière que les dimensions choisies permettent à l'individu d'utiliser ledit outil avec le minimum d'efforts pour le maximum d'effets.

## Description

La présente invention se rapporte aux dispositifs de mesure anthropométrique. Elle concerne tout particulièrement les dispositifs pour la détermination de caractéristiques relatives à l'ossature d'un individu.

Dans la suite de ta description, le mot outil se rapportera à un objet mécanique avec lequel l'individu interagit au moyen de ses mains et/ou de ses pieds.

Certains outils, tels qu'un vélo, par exemple, nécessitent un effort important de la part de l'individu qui l'utilise. L'efficacité de l'effort consenti varie considérablement selon que l'outil est ou non dimensionné de manière qu'il soit adapté à la morphologie de cet individu. Lors d'un usage prolongé de l'outil, des dimensions inadéquates peuvent provoquer des problèmes tels qu'inflammations des articulations, des ligaments ou des muscles, par exemple. Il est donc important de pouvoir adapter, de manière optimale, l'outil à son utilisateur.

Dans le cas du vélo, l'entreprise FSP Ferraroli de Lugano (Suisse) a développé un simulateur comportant, comme un vélo, un cadre, une selle, un guidon et un pédalier, et sur lequel un utilisateur potentiel est soumis à des tests, pour mesurer l'effort qu'il accomplit en pédalant dans des conditions données. Ce simulateur permet de modifier les conditions d'utilisation et tout particulièrement les dimensions du cadre, du guidon et du pédalier, ainsi que la position de la selle.

Le pédalier est relié à un système d'entraînement couplé à un dispositif électro-magnétique, qui mesure le travail fourni. Il comporte, en outre, des moyens pour mesurer des paramètres physiologiques permettant de déterminer l'effort consenti par l'utilisateur dans certaines conditions. Le rapport entre travail fourni et effort consenti détermine le rendement obtenu. Sur ce simulateur, la plupart des dimensions peuvent être modifiées, comme, par exemple, celles du cadre, la position et la largeur du guidon, la longueur de chacune des manivelles du pédalier, etc. Avec un tel simulateur, il est possible, par tâtonnement, de déterminer les dimensions les plus favorables pour un individu.

Avec le temps, l'homme du métier a montré des corrélations entre les dimensions de l'individu et celles de l'outil qu'il veut utiliser, structurées sous forme de tabelles ou de graphiques. De manière habituelle, l'homme du métier mesure, au moyen d'un ruban métrique, la hauteur de l'individu, la longueur de ses jambes et de ses cuisses, celle de ses bras et de son tronc. A partir de là et des tabelles mentionnées plus haut, il est possible de prérégler l'appareil de mesure de façon que le tâtonnement soit minimal.

La pratique a, toutefois, révélé que les individus présentent, très souvent, des dissymétries qui ne peuvent que difficilement être détectées par des mesures habituelles et qui contribuent largement aux problèmes de santé évoqués ci-dessus. Ces dissymétries apparaissent lors des essais sur le simulateur et conduisent à la réalisation d'un outil les compensant. Cela implique, toutefois, de nombreux tâtonnements, nécessitant beaucoup de temps, d'où une augmentation du coût de l'outil. Le dispositif selon l'invention permet de réduire considérablement ce temps. En outre, lorsque l'optimum n'est pas cherché, il est possible de réaliser un outil qui s'en approche de manière remarquable, pour un faible coût. Ce but est atteint grâce au fait que le dispositif selon l'invention comporte:
- un podium sur lequel l'individu prend place,
- des marques disposées sur le corps de cet individu, au moins en des endroits correspondant à la position d'articulations,
- des moyens de prise de vue de type numérique pour enregistrer les informations visibles concernant l'individu placé sur le podium, et
- un ordinateur muni d'un programme informatique agencé de manière à déterminer la structure de l'ossature et à calculer les dimensions d'un outil devant être manipulé par l'individu examiné,
le tout agencé de manière que les dimensions choisies permettent à l'individu d'utiliser cet outil avec le minimum d'efforts pour le maximum d'effets.

Les essais pratiques ont montré que les meilleurs résultats étaient obtenus lorsque l'individu concerné est examiné en position debout. Dans cette postion, les dimensions définies lors de la mesure permettent de réaliser un outil dont le rendement est voisin de 85% de l'optimum.

A cours des essais, il est apparu qu'avec une vue seulement, même de face, les informations pouvant être recueillies étaient trop limitées. Il serait, certes, possible de faire tourner l'individu et d'effectuer plusieurs prises de vues successives, selon des angles différents. Une telle solution ne donne pas satisfaction, car il est difficile d'établir une corrélation entre les différentes prises de vue.

C'est pourquoi, de façon avantageuse, le dispositif selon l'invention est agencé de manière à ce que l'individu se tienne debout sur le podium et porte des marques disposées sur son corps aussi dans des parties qui ne sont pas directement visibles de la position dans laquelle se trouve l'appareil de prise de vues. En outre, le dispositif comporte au moins un miroir, placé latéralement et derrière l'individu en référence à l'appareil de prise de vue, de manière à pouvoir saisir, au moyen de l'appareil de prise de vue, toutes les marques disposées sur son corps.

Il est, en particulier souhaitable que le dispositif comporte deux miroirs disposés symétriquement.

Lorsque l'outil à dimensionner est utilisé en position assise, comme c'est le cas d'un vélo, par exemple, le point d'appui que forme la selle joue un rôle important. Ce point d'appui peut difficilement être identifié, car il est masqué par les fesses. Pour pallier cet inconvénient, et dans une variante particulièrement intéressante, le dispositif comporte, en outre, une colonne munie de moyens de guidage verticaux, une barre de mesure horizontale, destinée à être disposée dans l'entrejambes, montée mobile verticalement sur ladite colonne et associée à des moyens pour engendrer une force orientée vers le haut, pour assurer que la barre hoizontale reste en permanence en contact avec le corps de l'individu.

Afin de faciliter la détermination de ce point d'appui, la barre est également munie d'une marque comparable à celles disposées sur le corps de l'individu.

Pour assurer une saisie des informations qui soit suffisamment précise et qui ne nécessite qu'un temps de calcul minimum, les moyens de prise de vues sont formés par un appareil photographique de type numérique. L'angle de prise de vue est particulièrement bien adapté lorsque la distance mesurée horizontalement entre l'appareil et l'individu est égale à environ 1,5 fois la hauteur de l'individu.

Dans le cas du vélo, l'essentiel de l'effort est fourni par les pieds travaillant sur les pédales. L'effort peut être dispensé dans de bonnes conditions dans la mesure seulement où les pieds trouvent un appui satisfaisant. Afin de permettre l'acquisition de connaissances relatives à ce paramètre, en même temps que se fait la saisie d'informations relative au corps de l'individu, le podium comporte une plaque transparente, destinée à servir d'appui aux pieds de l'individu, et un miroir disposé sous la plaque et renvoyant l'image du contact des pieds sur la plaque, vers l'appareil photographique.

D'autres avantages et caractéristiques de l'invention ressortiront de la description qui va suivre, faite en regard du dessin annexé, dans lequel:
- Les figures 1 et 2, montrent, respectivement vu de face légèrement décalé et de côté, un dispositif selon l'invention, dans lequel un individu est installé, et
- La figure 3 représente un schéma logique de ce dispositif.

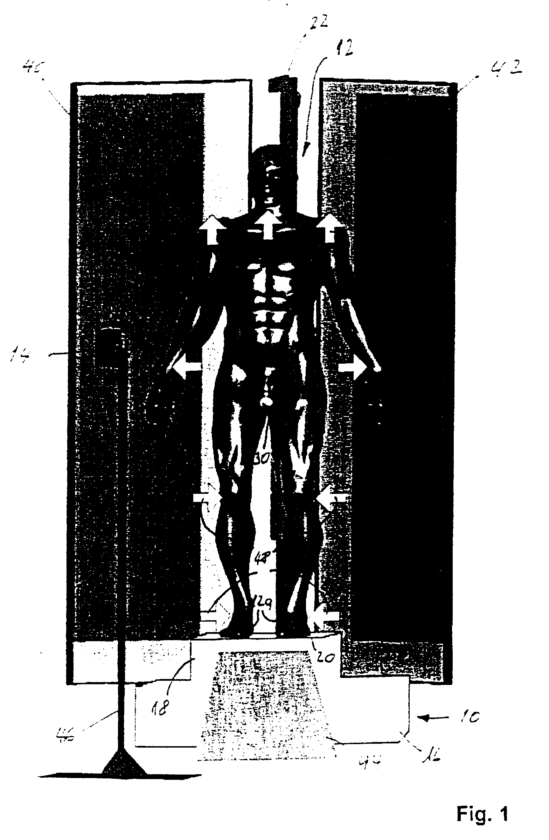

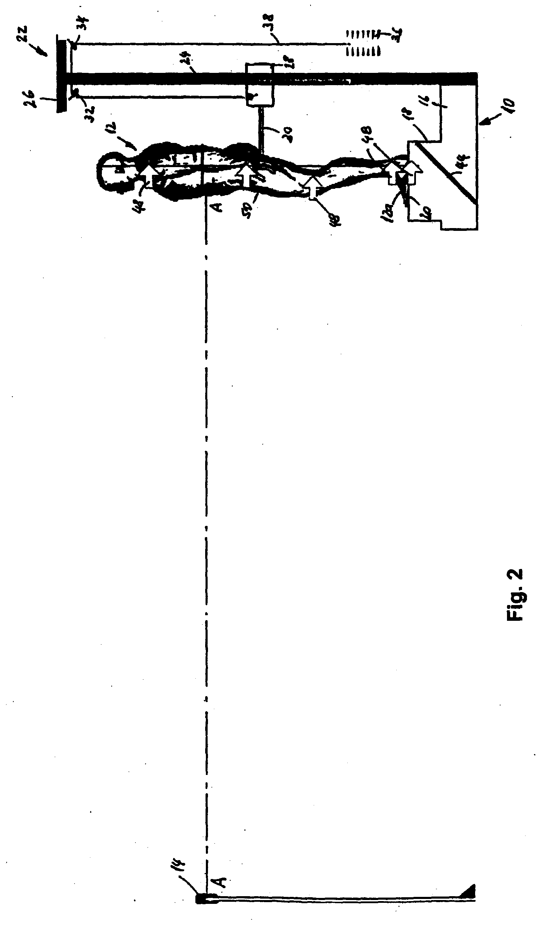

Le dispositif représenté aux figures 1 et 2 est destiné à des mesures anthropométriques pour la détermination de caractéristiques relatives à l'ossature d'un individu, dans le but de fabriquer un vélo ajusté à sa morphologie. Ce dispositif comporte un podium 10 destiné à recevoir successivement les individus objets de ces mesures, l'un d'eux étant schématiquement représenté en 12, et un appareil photographique 14.

Le podium 10 comporte une base 16, de forme générale rectangulaire, un piédestal 18, disposé sur la base 16 et dont la face supérieure est formée d'une plaque en verre 20, sur laquelle l'individu 12 se plante, ses pieds 12a étant totalement inscrits dans la surface de la plaque 20.

Une potence 22, formée d'un montant 24 et d'une traverse 26, est fixée à la base 16, derrière le piédestal 18. Elle sert de support à un chariot 28 monté coulissant sur le montant 24 et muni d'une barre horizontale 30 dont la fonction sera précisée plus loin. La traverse 26 porte deux poulies 32 et 34. Un contre-poids 36 est relié au chariot 28 par une courroie 38 passant par-dessus les poulies 32 et 34. Sa masse est supérieure à celle du chariot 28, de sorte que ce dernier tend à se déplacer vers le haut.

La base 16 porte, en outre, deux miroirs 40 et 42 disposés symétriquement, de part et d'autre de la potence 22, distants l'un de l'autre d'environ 30 cm, s'étendant sur une hauteur d'environ 2 m et chacun large d'environ 50 cm. Les surfaces de réflexion de ces miroirs définissent, entre elles, un angle compris entre 90° et 180°. De manière avantageuse, les miroirs 40 et 42 sont montés pivotants, autour d'axes verticaux, sur des supports solidaires de la potence 22, qui n'ont pas été représentés au dessin par souci d'éviter de le surchargé.

La base 16 et le piédestal 18 sont formés de caissons creux superposés, ouverts vers l'avant. Un troisième miroir 44 est disposé dans la base, en dessous du piédestal 18. Il est incliné d'environ 45° par rapport à la plaque de verre 20 et disposé de manière que l'appui des pieds sur cette dernière soit cadré par l'appareil photographique 14. Un système d'éclairage, non représenté, est également logé dans la base 16, placé de façon à éclairer la plaque de verre 20.

L'appareil photographique 14 est disposé sur un support 46 à l'avant du podium, à une distance environ égale à 1,5 fois la hauteur de l'individu 12 et à une hauteur telle qu'un axe horizontal A-A passant par l'objectif traverse le corps de l'individu 12 au niveau du sternum. Il est muni d'un objectif choisi de manière que l'image saisie embrasse non seulement l'individu, mais également les images renvoyées par les miroirs 40, 42 et 44.

Les opérations de mensuration comportent trois étapes:
- le marquage du corps de l'individu,
- la prise de vue, et
- le calcul des caractéristiques du vélo.

L'opération de marquage consiste à disposé des marques, schématiquement représentées par des flèches 48, faites par exemple avec de l'encre fluorescente, aux endroits correspondant aux points significatifs du corps, aussi bien à l'avant, à l'arrière que sur les côtés, directement sur le corps ou sur une combinaison moulante. Parmi ces endroits, on relèvera tout particulièrement les articulations des épaules et des poignets, des hanches, des genoux et des chevilles.

L'individu 12 prend, ensuite, place sur le podium 10, tourné vers l'appareil 14, ses pieds disposés sur la plaque de verre 20. La barre 30 est placée dans l'entrejambes, de manière qu'elle dépasse légèrement vers l'avant. Elle est munie d'une pastille 50 ayant des caractéristiques comparables à celles des marques 48. Elle est maintenue en place sous l'effet du contre-poids 36.

En éclairant l'individu 12 avec une source de lumière ultra-violette de type traditionnelle, les marques 48 et la pastille 50 apparaissent de manière particulièrement contrastée sur l'image du corps.

L'image, saisie par l'appareil photographique 14, comporte une vue de face de l'individu, deux vues des côtés et de l'arrière, renvoyées par les miroirs 40 et 42 et une vue du contact des pieds renvoyée par le miroir 44. Ces images sont définies par les points que forment la matrice des capteurs photoélectriques de l'appareil photographique numérique. Chaque point peut donc être caractérisé par une coordonnée, dans un référentiel à trois dimensions, la profondeur étant définie par les images renvoyées par les miroirs 40 et 42.

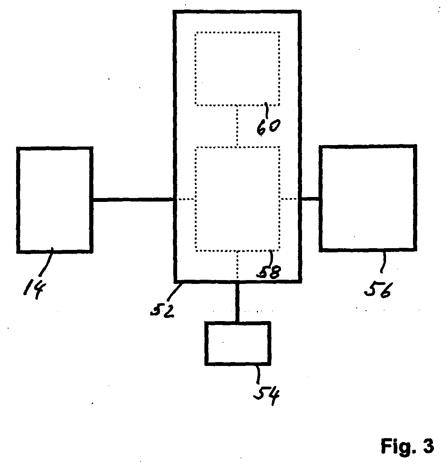

Pour traiter ces images, te dispositif comporte des moyens de calcul schématiquement représentés sur la figure 3 et qui comprennent essentiellement un ordinateur 52 muni d'un clavier de commande 54 et d'une imprimante 56. L'ordinateur 52 comporte, en particulier, une unité de calcul 58 et une mémoire 60 dans laquelle se trouve le logiciel de commande, Il est relié à l'appareil 14, directement ou par l'intermédiaire d'une disquette contenant, sous forme binaire, les informations relatives aux images prises.

Dans un premier temps, l'ordinateur 52 calcule, à partir de la vue de face et des vues latérales renvoyées par les miroirs 40 et 42, c'est-à-dire à partir des coordonnées des points considérés, les paramètres de l'ossature de l'individu 12. Il détermine tout particulièrement la longueur des bras, des tibias et des fémurs, les distances entre les hanches et les épaules, tant pour le côté droit que pour le côté gauche. De la sorte, il est possible de mettre en évidences d'éventuelles dissymétries.

Les paramètres de l'ossature étant connus, il est alors facile de les introduire dans les tabelles définies à partir des résultats obtenus au moyen de l'appareil commercialisé par l'entreprise FSP Ferraroli mentionnée plus haut, et qui établissent les corrélations entre les paramètres de l'ossature et les mesures des organes du vélo.

Ainsi, les dimensions du tronc et des bras définissent l'espace entre la selle et le guidon, tandis que la longueur des jambes détermine la distance entre la selle et les pédales. Plus précisément, les jambes définissent la hauteur de la selle, alors que le rapport des longueurs du fémur et du tibia permet de calculer l'inclinaison du tube vertical du cadre.

Les dimensions calculées peuvent ensuite être introduites dans le simulateur afin d'optimiser le vélo. Elles peuvent aussi être rendues lisibles au moyen de l'imprimante 56 et introduites manuellement.

Lorsque le niveau d'exigence est plus faible, correspondant sensiblement à environ 85% de l'optimum, le mécanicien peut fabriquer un vélo correspondant au mieux à la morphologie de l'individu 12 sur la base des seules données définies par l'imprimante. En variante, ces données peuvent être directement introduites dans une machine de type à commande numérique, préprogrammée et qui peut directement fabriquer les pièces aux dimensions idoines.

L'image renvoyée par le miroir 44 permet de déterminer si l'assise de l'individu est satisfaisante, ou si la voûte plantaire de l'un et/ou de l'autre pied pose problème et nécessiterait un support.

Il va de soi que les prises de vues, ou les paramètres déterminés par l'ordinateur, sont conservés en mémoire, de sorte qu'il est possible de suivre, au cours du temps, l'évolution de la morphologie d'une personne et, si nécessaire, adapter le vélo en fonction des modifications enregistrées.

Dans le mode de réalisation décrit ci-dessus, le dispositif permet de dimensionner un vélo de manière particulièrement efficace et pour un coût modeste. Un tel dispositif peut également être utilisé dans d'autres domaines sportifs, pour le choix d'un équipement de ski de fond par exemple, mais aussi pour l'adaptation d'outils, d'appareils ou de machines dont l'usage nécessite un effort physique, par exemple dans le domaine du génie civil ou du bâtiment.

## Revendications

1. Dispositif de mesure anthropométrique pour la détermination de caractéristiques relatives à l'ossature d'un individu (12), caractérisé en ce que ledit dispositif comporte:
• un podium (10) sur lequel ledit individu (12) prend place,
• des marques (48) disposées sur le corps dudit individu (12), en des endroits correspondant à la position d'articulations,
• des moyens de prise de vue (14) de type numérique pour enregistrer les informations visibles concernant l'individu placé sur le podium,
• un ordinateur (52) muni d'un programme informatique agencé de manière à déterminer la structure de l'ossature et à calculer les dimensions d'un outil devant être manipulé par ledit individu,
le tout agencé de manière que les dimensions choisies permettent à l'individu d'utiliser ledit outil avec le minimum d'efforts pour le maximum d'effets.

2. Dispositif selon la revendication 1, caractérisé en ce qu'il est agencé de manière à ce que l'individu (12) se tienne debout sur le podium (10) et en ce qu'il porte des marques (48) disposées sur son corps aussi dans des parties qui ne sont pas directement visibles de la position dans laquelle se trouve l'appareil de prise de vues (14), et au moins un miroir (40, 42), placé latéralement et derrière ledit individu (12) en référence à l'appareil de prise de vue (14), de manière à pouvoir saisir, au moyen dudit appareil de prise de vue, toutes les marques disposées sur son corps.

3. Dispositif selon la revendication 2, caractérisé en ce qu'il comporte deux miroirs (40, 42) disposés symétriquement.

4. Dispositif selon l'une des revendications 2 et 3, caractérisé en ce qu'il comporte, en outre, une colonne (22) munie de moyens de guidage verticaux, une barre de mesure horizontale (30), destinée à être disposée dans l'entrejambes, montée mobile verticalement sur ladite colonne (22) et associée à des moyens (36) pour engendrer une force orientée vers le haut, pour assurer que ladite barre (30) reste en permanence en contact avec le corps de l'individu (12).

5. Dispositif selon la revendication 4, caractérisé en ce que ladite barre (30) est également munie d'une marque (50) comparable à celles (48) disposées sur ledit corps.

6. Dispositif selon l'une des revendications 2 à 5, caractérisé en ce que lesdits moyens de prise de vues sont formés par un appareil photographique (14) de type numérique disposé à une distance mesurée horizontalement égale à environ 1,5 fois la hauteur de l'individu.

7. Dispositif selon la revendication 6, caractérisé en ce que ledit podium (10) comporte une plaque transparente (20) destinée à servir d'appui aux pieds de l'individu et en ce qu'un miroir (44) est disposé sous la plaque et renvoie l'image du contact des pieds sur la plaque vers l'appareil photographique (14).
